# EUROPEAN PATENT SPECIFICATION

(11) **EP 3 978 922 B1**
(45) Date of publication and mention of the grant of the patent: **07.06.2023**
(21) Application number: 20200043.6
(22) Date of filing: 05.10.2020
(51) Int. Cl.: G01N 33/487, A01N 1/02, A61B 50/00, A61B 50/20, B01L 9/06

(54) **PORTABLE PROTECTIVE BOX FOR A MEDICAL FLASK**
TRAGBARER SCHUTZKASTEN FÜR EIN MEDIZINISCHES FLÄSCHCHEN
BOÎTE DE PROTECTION PORTABLE POUR UN FLACON MÉDICAL

(43) Date of publication of application: 06.04.2022
(60) Divisional application: 23161731.7
(73) Proprietor: GWA Hygiene GmbH, 18435 Stralsund (DE)
(72) Inventor: WALZ, Marcel, 18437 Stralsund (DE); GEBHARDT, Tobias, 18119 Rostock (DE)
(74) Representative: Röthinger, Rainer

(56) References cited:
- EP-A1- 2 668 847
- WO-A1-2017/158579
- WO-A1-2019/198028
- CN-U- 207 985 571
- CN-U- 208 790 176
- US-A1- 2017 320 054

## Description

### Technical Field

The present disclosure generally relates to a portable protective box for a medical flask and a system comprising the box.

### Background

In certain environments, hazardous medical liquids or medical liquids to be protected from impurities are temporarily stored in medical flasks. Such medical flasks may contain a serum and a blood sample of a patient. For instance, when testing a patient for sepsis, blood samples are taken of the patient and injected into different medical flasks, each containing a different serum. In a medical laboratory, the medical flasks may be placed in a breeding chamber for a certain amount of time. Afterwards, the flask's contents may be screened for certain bacteria or microbes. Usually, test kits comprising a predefined amount of different medical flasks (e.g., six flasks) containing different serums are used when testing a patient for sepsis, and the size of the blood sample to be injected into each medical flask must exceed a minimum volume for the testing to yield reliable results.

As another example, blood samples of a patient may be taken using one or more syringes as medical flasks, which then need to be transported to the medical laboratory. In this case, a blood analysis may be conducted in the medical laboratory, for example to determine hormone levels, a concentration of glucose or a concentration of a certain type of blood cells. Also in this case, a set of (e.g., differently sized) syringes filled with blood samples may be required to enable a complete blood analysis, and the size of the blood samples in the syringes may need to exceed a minimum volume for the blood analysis to yield reliable results.

Sometimes, medical staff may forget to use all of the required medical flasks (e.g., all flasks of the test kit or all syringes of the set of syringes), or accidentally fill one of the medical flasks with a blood sample having an insufficient volume. In other cases, the medical flasks of different test kits or the syringes of different sets of syringes may accidentally be swapped. Such errors may only be detected once the medical flasks reach the laboratory or even later (e.g., after testing), if at all.

In both of the above cases, the medical flasks may need to be safely transported between a location of the patient and the medical laboratory. In certain hospitals, the medical flasks are transported using a pneumatic mail system. Currently, the medical flasks are transported either in an open paper basket or randomly wrapped in bubble wrap or other plastic sheets for protection. Such packaging may not sufficiently protect the medical flasks so that a transported flask may break. Especially in case the broken flask contained a blood sample, no complete tests may be able to be carried out in the medical laboratory, or the test results may be negatively affected. Even further, contents leaking from the broken medical flask may contaminate the environment (e.g., with multiple resistant staphylococcus aureus, MRSA).

US 2017/320054 A1 discloses an assembly for transporting a tissue sample that has been removed from a subject. The assembly includes a transport container, a fixative in the transport container, and a cooling device that reduces and/or maintains the temperature of the fixative to perform a pre-soaking process at a temperature of less than about 7° C.

CN 208 790 176 U discloses a substrate box assembly including: a substrate bottle for holding substrate liquid, a box cover and a shell. The box cover is arranged on the side of the shell, and the box cover and the shell are rotatably connected, and the inside of the shell forms an accommodating space for accommodating the substrate bottle. A sensor component for detecting the remaining amount of the substrate liquid in the substrate bottle is provided and a base component for placing the substrate bottle and the sensor component. The weight of the substrate bottle in the substrate box assembly is directly read by the sensor component.

CN 207 985 571 U discloses an intelligent medicine box including a medicine box body, a plurality of medicine bottle grooves, a PCB board, a battery, a LED pilot lamp, a sensor and wireless communication module. A medicine bottle is placed in a medicine bottle groove, and the LED pilot lamp is used to instruct a user which medicine to take.

WO 2017/158579 A1 discloses a system for transporting goods comprising one or more containers which are adapted to contain goods, which containers communicate with a remote unit. Each container has a housing compartment for goods, which housing compartment is delimited by one or more walls and has at least one opening, which opening is closed by a cover element. The cover element comprises at least one sensor for detecting the accelerations acting on the container, said cover element having at least one transmit/receive unit for communication with said remote unit.

WO 2019/198028 A1 discloses a case for packaging and monitoring biological samples, comprising: a body with an internal space for storing the samples and an upper lid for closing the body; spaces for receiving a plurality of bases with identified biological samples; at least one temperature sensor for sensing the temperature profile during transport; at least one accelerometer for sensing vibrations during the sample transport; a data processor programmed to send within a predefined time interval the temperature and vibration Information captured by the sensors; at least one radio frequency identification antenna to communicate the information captured to the plurality of bases, wherein each base is capable of storing and transmitting the information sent by the antenna.

EP 2 668 847 A1 discloses a container for carrying biological samples, comprising a location detection device and an inertial sensor for the position of the container, and a control unit which comprises a communications device which is adapted to transmit the data that arrive from the location detection device and from the inertial position sensor to a central unit, the control unit comprising a device for storing the data.

### Summary

There is a need for a box for a medical flask that solves one or more of the aforementioned or other problems.

According to a first aspect, a portable protective box for a medical flask is provided. The box comprises a housing and a flask holder encased by the housing and fixed relative to the housing, the flask holder comprising a retainer configured to retain a medical flask in a stationary position relative to the housing. The box further comprises at least one sensor element having a predefined position relative to the retainer and being configured to enable detection, when the medical flask is retained by the retainer, of at least one of the medical flask being retained by the retainer, a filling level of the medical flask, a material of the medical flask and a type of fluid inside the medical flask. The at least one sensor element comprises at least one capacitance measurement electrode. The at least one capacitance measurement electrode comprises a first electrode segment and a second electrode segment contacting the first electrode segment, wherein the first electrode segment is elongated in a first direction of the retainer and the second electrode segment is elongated in a second direction of the retainer different from the first direction.

In one example, the first direction is essentially perpendicular to the second direction. Alternatively or additionally, the first direction may be a longitudinal direction of the retainer and the second direction may be a circumferential direction of the retainer.

The box in one variant further comprises processing circuitry connected to the at least one sensor element. The processing circuity is configured to obtain a sensor signal using the at least one sensor element, generate, based on the sensor signal, information indicative of at least one of the medical flask being retained by the retainer, a filling level of the medical flask when the medical flask is retained by the retainer, a material of the medical flask when the medical flask is retained by the retainer, and a type of fluid inside the medical flask when the medical flask is retained by the retainer.

The sensor signal may be representative of a capacitance value measured using the at least one capacitance measurement electrode. In one example, the processing circuitry is configured to measure a first capacitance value using a first of the at least one capacitance measurement electrode, measure a second capacitance value using a second of the at least one capacitance measurement electrode, and generate the information based on the first capacitance value and the second capacitance value. The first of the at least one capacitance measurement electrode may have a different geometry than the second of the at least one capacitance measurement electrode.

The box for example further comprises a flexible circuit board, wherein the at least one capacitance measurement electrode is arranged on the flexible circuit board and the flexible circuit board is formed such that the at least one capacitance measurement electrode conforms to a shape of at least one of the retainer and, when the medical flask is retained by the retainer, the medical flask.

The box may further comprise a receiver configured to receive a wireless signal, wherein the processing circuitry is configured to obtain, from the received wireless signal, information indicative of at least one of an identity of a sender of the wireless signal and a location of the box.

In one example, the at least one sensor element comprises at least one of a light detection element and a pressure detection element.

The flask holder may comprise a bottom portion and a top portion separable from the bottom portion, wherein a first part of the retainer is formed in the bottom portion and a second part of the retainer is formed in the top portion.

For example, the flask holder comprises a plurality of the retainers, each retainer being configured to retain a separate medical flask in a stationary position relative to the housing, wherein the box comprises a plurality of the at least one sensor element, each of the at least one sensor element having a predefined position relative to a different one of the plurality of the retainers.

The box may further comprise a set of arms tensioned in an outward direction of the box, the arms being configured to hold the box inside a transport capsule of a pneumatic post system when the box is inserted into the transport capsule. According to a second aspect, a system is provided. The system comprises the box of the first aspect and a transport capsule of a pneumatic post system, the transport capsule having an interior storage room accessible from an outside of the capsule through a closable opening, wherein the housing of the box is shaped such that it can be completely inserted into the storage room through the opening.

According to a third aspect, a system is provided comprising the box of the first aspect or the system of the second aspect, wherein the flask holder is removably attached to the housing and the at least one sensor element. The system further comprises at least one replacement holder removably attachable to the housing when the flask holder is detached from the housing, the at least one replacement holder comprising a retainer configured to, when the replacement holder is removably attached to the housing, retain a medical flask in a stationary position relative to the housing, wherein the retainer of the replacement holder has a different geometry or orientation than the retainer of the flask holder.

In one example, when the flask holder is detached from the housing and the replacement holder is removably attached to the housing, the at least one sensor element has a predefined position relative to the retainer of the replacement holder and is configured to enable detection of at least one of the medical flask being retained by the retainer of the replacement holder, a filling level of the medical flask, a material of the medical flask and a type of fluid inside the medical flask when the medical flask is retained by the retainer of the replacement holder.

### Brief Description of the Drawings

Further details, advantages and aspects of the present disclosure will become apparent from the following embodiments taken in conjunction with the drawings, wherein:
- Fig. 1: shows a perspective view of an exemplary embodiment of a portable protective box in accordance with the present disclosure;
- Fig. 2: shows a perspective view of the exemplary embodiment of Fig. 1 with a plurality of inserted medical flasks;
- Fig. 3: shows a first perspective view of the exemplary embodiment of Fig. 1 with a lower housing part removed;
- Fig. 4: shows a second perspective view of the exemplary embodiment of Fig. 1 with the lower housing part removed;
- Fig. 5: shows a perspective view of an exemplary embodiment of a flexible circuit board in accordance with the present disclosure;
- Fig. 6: shows a perspective view of an exemplary embodiment of a flexible circuit board in accordance with the present disclosure;
- Fig. 7: shows a top view of an exemplary embodiment of a portable protective box in accordance with the present disclosure;
- Fig. 8: shows a perspective view of an exemplary embodiment of parts of a portable protective box in accordance with the present disclosure;
- Fig. 9: shows a perspective view of an exemplary embodiment of a system in accordance with the present disclosure;
- Fig. 10: shows a schematic illustration of a system in accordance with the present disclosure;
- Fig. 11: shows an exemplary sequence of method steps performed by processing circuitry in accordance with the present disclosure;
- Fig. 12: shows an exemplary dataset in accordance with the present disclosure; and
- Fig. 13: shows an exemplary dataset in accordance with the present disclosure.

### Detailed Description

In the following description, exemplary embodiments of a portable protective box, a system and a method will be explained with reference to the drawings. The same reference numerals will be used to denote the same or similar structural features.

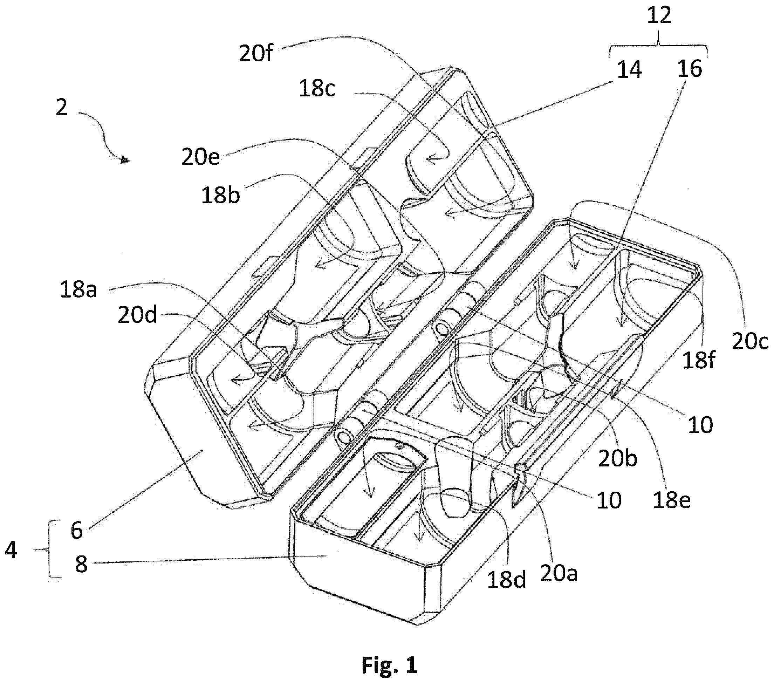

Fig. 1 shows a perspective view of an exemplary embodiment of a portable protective box 2 in accordance with the present disclosure. The box is relatively lightweight to be portable by a human without aid (e.g., weighs below 4 kg or below 2 kg). The box 2 comprises a housing 4 having an upper housing part 6 and a lower housing part 8. The upper housing part 6 is movably attached relative to the lower housing part 8. Note that the upper housing part 6 may be removably attached relative to the lower housing part 8. In the example of Fig. 1, the housing 4 comprises hinges 10 rotatably connecting the upper housing part 6 to the lower housing part 8. In a closed configuration, the upper housing part 6 is fixed relative to the lower housing part 8 and in an opened configuration, as shown in Fig. 1, the upper housing part is movable (e.g., rotatable) relative to the lower housing part 8. To fix the upper housing part 6 relative to the lower housing part 8 in the closed configuration, one or both of the upper housing part 6 and the lower housing part 8 may comprise an attachment member such as a latch, a fastener or else. In the example shown in Fig. 1, the attachment member is provided at a side of the housing 4 that is opposite to the hinges 10. To provide protection for a medical flask, the housing is made of a rigid material. Preferably, the rigid material cannot be manually deformed. The material may be polypropylene, unplasticized polyvinylchloride (PVC-U), polyamide or else.

The box 2 comprises a flask holder 12 encased by the housing 4 and fixed relative to the housing 4. When the housing 4 is in the closed configuration, the flask holder 12 is completely encased by the housing 4 such that it is not accessible from an outside of the housing 4. For example, the flaks holder 12 is removably attached to the housing 4. In the shown example, the flask holder 12 comprises a top (e.g., upper) portion 14 and a bottom (e.g., lower) portion 16. The top portion 14 is separable from the bottom portion 16. The top portion 14 is fixed relative to the upper housing part 6 and the bottom portion 16 is fixed relative to the lower housing part 8.

The flask holder 12 comprises a plurality of retainers, each configured to retain a medical flask in a stationary position relative to the housing 4. In the example of Fig. 1, each of the retainers is formed of a first part 18a-18f and a second part 20a-20f (e.g., one retainer is formed by (e.g., consists of) the first part 18a and the second part 20a, another retainer is formed by the first part 18b and the second part 20b and so on). The second part 20a-20f may be smaller (e.g., in at least one of volume and depth) than the first part 18a-18f. It is noted that one or more of the retainers may be formed of only the first part 18a-18f, i.e., may not comprise the second part 20a-20f. In the opened configuration, the retainers are accessible from an outside of the housing 4.

The top portion 14 comprises half of a total number of the first parts 18a-18f and the bottom portion 16 comprises the other half of the total number of first parts 18a-18f. As can be seen, the flask holder 12 comprises a total of six retainers, wherein the top portion 14 comprises the three first parts 18a-18c and the bottom portion 16 comprises the three first parts 18d-18f. The top portion 14 comprises the three second parts 20d-20f and the bottom portion 16 comprises the three second parts 20a-20c.

The retainers each are shaped to essentially match an outer contour of a (e.g., different) medical flask. The medical flask may be a blood culture bottle, a test tube, a syringe or else. The medical flask may have a volume of less than 100ml, for example between 5ml and 50ml. The medical flask may be essentially rotationally symmetric. The medical flask may comprise at least two portions having different diameters.

Each retainer may be formed as a slot in the flask holder 12, such that separate medical flasks can be inserted into different slots. For example, each retainer or slot has a same geometry. The retainers or slots may be arranged to provide a highest possible packing density inside the housing 4. Some of the retainers or slots may differ in their orientation relative to the housing 4. In the example shown in Fig. 1, the top portion 14 comprises the first parts 18b and 18c having a first orientation and the first part 18a having an opposite second orientation. In difference thereto, the bottom portion 16 comprises the first parts 18d and 18e having the second orientation and the first part 18f having the opposite first orientation.

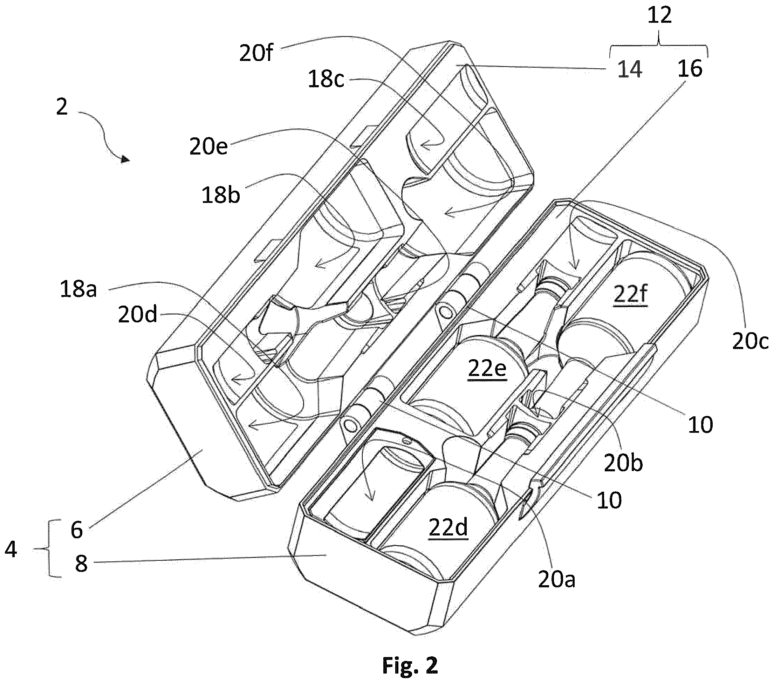

Fig. 2 shows a perspective view of the exemplary embodiment of Fig. 1 with a plurality of medical flasks 22d-22f. The medical flask 22d is inserted in the first part 18d, the medical flask 22e is inserted in the first part 18e and the medical flask 22f is inserted in the first part 18f. When the housing 4 is in the closed configuration, the medical flask 22d is retained by the retainer comprising the first part 18d and the second part 20d, the medical flask 22e is retained by the retainer comprising the first part 18e and the second part 20e and the medical flask 22f is retained by the retainer comprising the first part 18f and the second part 20f. Similarly, medical flasks (not shown) may be inserted in the first parts 18a-18c of the top portion 14 and may be retained in the retainers formed by the first parts 18a-18c and the second parts 20a-20c when the housing is in the closed configuration.

The box 2 allows for safely storing medical flasks (e.g., the medical flasks 22d-22f) inside the housing 4. In particular, the retainers reliably hold the medical flasks in a stationary position relative to the housing 4. Thus, even if the housing 4 is shaken or dropped onto a floor, the medical flasks inserted in the retainers may not significantly move relative to the flask holder 12. This may prevent the medical flasks from breaking.

Furthermore, a total number of the retainers may be identical to a total number of medical flasks comprised in a test kit (e.g., for testing sepsis) or a total number of syringes comprised in a set of syringes (e.g., for performing a complete blood analysis). In this case, the flask holder 12 guides the user on how many medical flasks are required by a medical laboratory. This may prevent the user from sending an insufficient number of medical flasks to the laboratory.

Also, the retainers may be formed so as to comply with an outer contour of a medical flask to be retained by the retainers. In this case, the user is guided which type of medical flasks to insert into which retainer. This may prevent the user from inserting a medical flask into a wrong retainer that does not match the medical flask's form, and, consequently, may prevent the user from sending wrongly formed medical flasks to the medical laboratory (e.g., sending cylindrical test tubes with a homogeneous diameter instead of blood culture flasks with a varying diameter as indicated in Fig. 2).

The box 2 further comprises at least one sensor element being associated with one of the retainers by having a predefined position relative to the one of the retainers (e.g., comprising the first part 18d and the second part 20d) and by being configured to enable detection, when the medical flask (e.g., the medical flask 22d) is retained by the one of the retainers, of at least one of the medical flask being retained by the one of the retainers, a filling level of the medical flask, a material of the medical flask and a type of fluid inside the medical flask. The box 2 may comprise a separate one of the at least one sensor element for each of the retainers, that is, the box 2 may comprise a plurality of the at least one sensor element, each associated with a different one of the retainers.

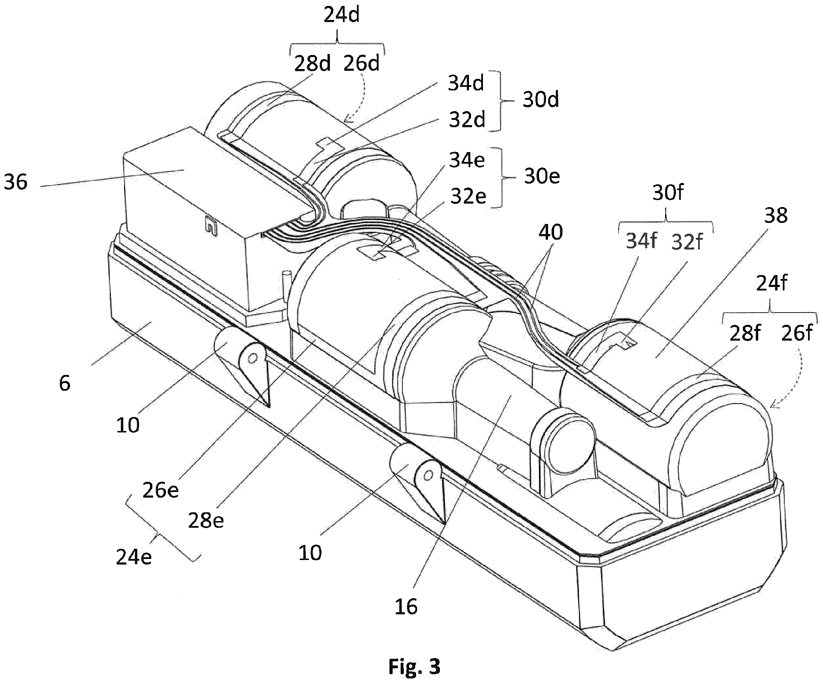

Fig. 3 shows a perspective view of the box 2 with the lower housing part 8 removed. As defined in claim 1, the at least one sensor element comprises at least one capacitance measurement electrode 24d-24f, 30d-30f. Although other shapes of the at least one capacitance measurement electrode 24d-24f, 30d-30f are possible, the at least one capacitance measurement electrode 24d-24f, 30d-30f shown in Fig. 3 is angled and may be referred to as having an "L"-form.

A first 24e of the at least one capacitance measurement electrode comprises a first electrode segment 26e and a second electrode segment 28e contacting the first electrode segment 26e, wherein the first electrode segment 26e is elongated in a first direction of the retainer comprising the first part 18e and the second electrode segment 28e is elongated in a second direction different from the first direction. The first electrode 24e may be referred to as being associated with the retainer comprising the first part 18e or as being arranged at the retainer comprising the first part 18e.

A second 30e of the at least one capacitance measurement electrode comprises a first electrode segment 32e and a second electrode segment 34e contacting the first electrode segment 32e, wherein the first electrode segment 32e is elongated in the first direction of the retainer comprising the first part 18e and the second electrode segment 34e is elongated in the second direction different from the first direction.

The second electrode 30e may be referred to as being associated with the retainer comprising the first part 18e or as being arranged at the retainer comprising the first part 18e.

The first direction may be parallel or orthogonal to a flat portion of an outer shape of the housing 4, such as a flat portion on which the box 2 can rest when the box 2 rests on a horizontal plane such as a table top. The first direction may be parallel to a surface of liquid inside the medical flask retained by the associated retainer when the box 2 rests on the horizontal plane. The first direction may be (e.g., essentially) perpendicular to the second direction. In the example shown in Fig. 3, the first direction is a longitudinal direction of the retainer, whereas the second direction is a circumferential direction of the retainer.

The first electrode segment 26e is spaced apart from the first electrode segment 34e in the circumferential direction of retainer associated to the electrode 24e, for example between 45 and 90 degrees, preferably approximately 90 degrees. The second electrode segment 28e is spaced apart from the second electrode segment 34e in the longitudinal direction of the associated retainer. The first electrode segment 26e is longer in the longitudinal direction of the associated retainer than the first electrode segment 32e. The second electrode segment 28e is longer in the circumferential direction of the associated retainer than the second electrode segment 34e. The (e.g., second electrode segment of the) first 24e of the at least one capacitance measurement electrode (e.g., the second electrode segment 28e) may be arranged at a first longitudinal end of a deepest or broadest section of the associated retainer. The (e.g., second electrode segment of the) second 30e of the at least one capacitance measurement electrode (e.g., the second electrode segment 34e) may be arranged at a second longitudinal end of the deepest or broadest section of the associated retainer. The first electrode segment 26e may extend between the first longitudinal end and the second longitudinal end. The first longitudinal end may be spaced apart from the second longitudinal end in the longitudinal direction of the associated retainer.

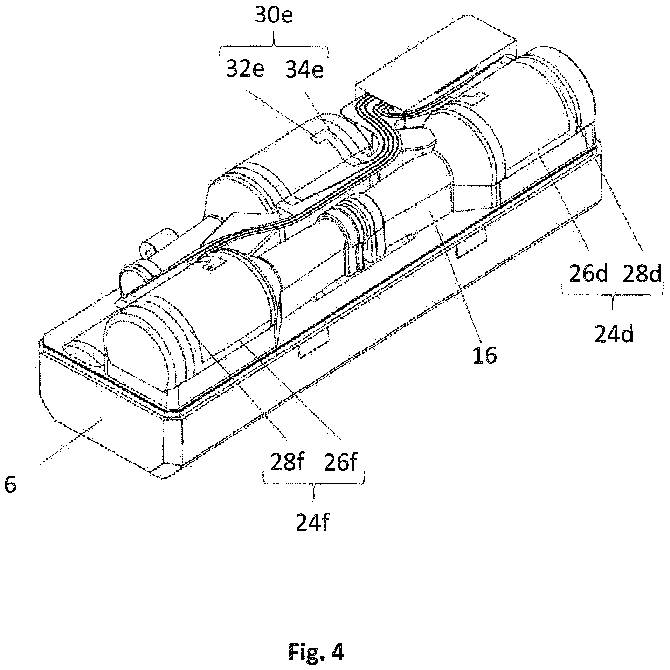

Additional instances of the at least one capacitance measurement electrode may be arranged at the other retainers. In other words, the box 2 may comprise a plurality of the at least one capacitance measurement electrode, wherein each of the plurality of the at least one capacitance measurement electrode is associated to a different retainer. In the example of Fig. 3, and as apparent from Fig. 4 showing a different perspective view of the box 2 of Fig. 3, the at least one measurement electrode is also provided for the retainer comprising the first part 18d and the retainer comprising the first part 18f, as indicated with reference signs 24d, 24f for the first of the at least one capacitance measurement electrode, reference signs 30d, 30f for the second of the at least one capacitance measurement electrode, reference signs 26f, 26f for the first electrode segment of the electrodes 24d, 24f, reference signs 28d, 28f for the second electrode segment of the electrodes 24d, 24f, reference signs 32d, 32f for the first electrode segments of the electrodes 30d, 30f and reference signs 34d, 34f for the second electrode segments of the electrodes 30d, 30f. Of course, additional instances of the at least one measurement electrode may also be associated with the retainers comprising the first parts 18a-18c (not shown in Figs. 3 and 4).

The first 24d-24f of the at least one capacitance measurement electrode may be arranged such that it extends approximately along a height of a liquid level inside the medical flask 22d-22f retained by the associated retainer, when the box 2 is oriented such that either the lower housing part 8 or the upper housing part 6 rests on a table. With such a configuration, it may be possible to detect a filling level of the medical flask 22d-22f irrespective of an orientation of the box 2, at least for filling levels above 50%. The shown arrangement of the second 30d-30f of the at least one capacitance measurement electrode may enable determining an orientation of the box 2 based on a capacitance measurement of the second 30d-30f of the at least one capacitance measurement electrode (e.g., and further based on a capacitance measurement of the first 24d-24f of the at least one capacitance measurement electrode) in case an incompletely filled medical flask 22d-22f is retained by the associated retainer.

The flask holder 12 in this example comprises a compartment 36 configured to house processing circuitry (not shown). The electrodes 24d-24f and 30d-30f are arranged on a flexible circuit board 38. The flexible circuit board 38 comprises a plurality of electrical connections 40, each connecting one of the electrodes 24d-24f, 30d-30f to the processing circuitry. The flexible circuit board 38 may be a printed circuit board. The flexible circuit board 38 is formed such that each of the at least one capacitance measurement electrode 24d-24f, 30d-30f (e.g., the electrode 24d) conforms to a shape of at least one of the associated retainer (e.g., the retainer comprising the first part 18d) and, when the medical flask 22d-22f (e.g., the medical flask 22d) is retained by the associated retainer, the medical flask 22d-22f.

For example, the flask holder 12 comprises a wall section (not shown in Figs. 3 and 4 as it lies beneath the flexible circuit board 38 in these illustrations) having a front side forming at least a part of the associated retainer (e.g., the first part 18a-18f) and having an opposite back side, wherein the flexible circuit board 38 is formed such that each of the at least one capacitance measurement electrode 24d-24f, 30d-30f conforms to (e.g., matches) the back side of the wall section. The front side of the wall section may form the first part of the retainer. The wall section may have an essentially homogeneous thickness between the front side and the opposite back side.

By arranging the at least one capacitance measurement electrode 24d-24f, 30d-30f on the flexible circuit board 38, manufacturing costs can be reduced. Furthermore, it can be ensured that the at least one capacitance measurement electrode 24d-24f, 30d-30f has a form suitable for providing the intended measurement functions. In particular, the at least one capacitance measurement electrode 24d-24f, 30d-30f may be formed so as to conform to an outer contour of the medical flask 22d-22f inserted into the associated retainer. The flexible circuit board 38 may be deformed upon insertion of the medical flask into the associated retainer such that, after the deformation, the at least one capacitance measurement electrode 24d-24f, 30d-30f matches an outer contour of the medical flask 22d-22f inserted into the associated retainer (e.g., when the housing 4 is in the closed configuration). The box may comprise two flexible circuit boards 38, one being arranged in the upper housing part 6 and the other in the lower housing part 8, and each comprising a separate instance of the at least one sensor element.

Due to the shape of the at least one capacitance measurement electrode 24d-24f, 30d-30f described above, its capacity may change upon insertion of a medical flask 22d-22f into the associated retainer. Furthermore, the capacitance of the so-shaped at least one capacitance measurement electrode 24d-24f, 30d-30f may differ for a certain medical flask 22d-22f retained by the associated retainer, depending on a filling level of the retained medical flask 22d-22f. Still further, the capacitance of the at least one capacitance measurement electrode 24d-24f, 30d-30f may depend on a material of the medical flask 22d-22f retained in the associated retainer and a type of material (e.g., liquid) inside the medical flask 22d-22f retained in the associated retainer. Thus, when using the at least one capacitance measurement electrode 24d-24f, 30d-30f, one may obtain information related to the medical flask 22d-22f, as will also be discussed below with reference to the processing circuitry.

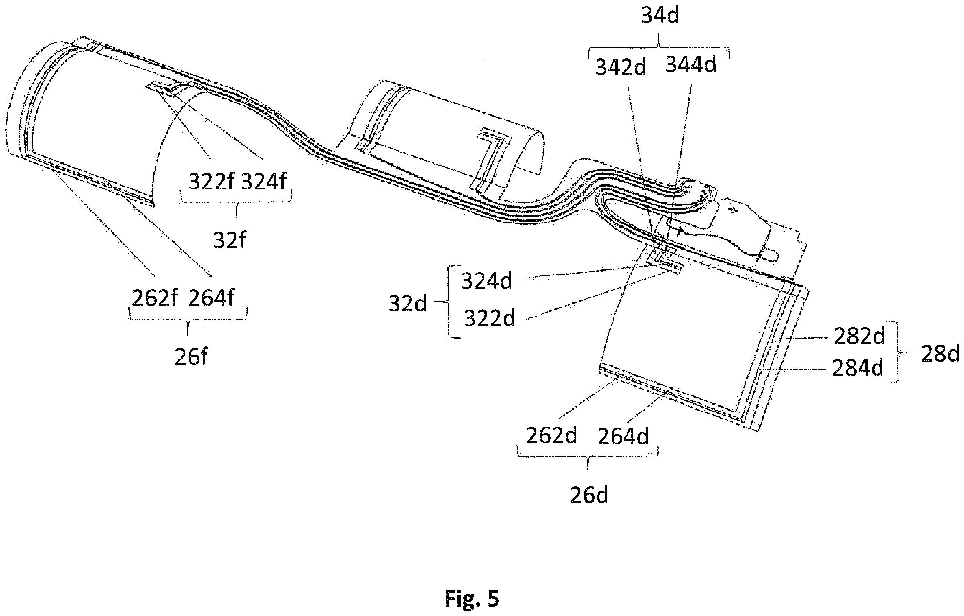

Fig. 5 shows an exemplary embodiment of a flexible circuit board. The flexible circuit board may be the flexible circuit board 38 shown in Figs. 3 and 4 and be comprised in the box 2. The flexible circuit board 38 shown in Fig. 5 comprises the electrodes 24d-24f, 30d-30f. In this example, each of the capacitance measurement electrodes 24d-24f, 30d-30f is formed by two parallel conductive traces. A portion of a first of the conductive traces forming the first electrode segment 26d is denoted with reference sign 262d, and a portion of a second of the conductive traces forming the first electrode segment 26d is denoted with reference sign 264d. Another portion of the first of the conductive traces forming the second electrode segment 28d is denoted with reference sign 282d and another portion of the second of the conductive traces forming the second electrode segment 28d is denoted with reference sign 284d. A similar splitting of the electrodes 24e-24f, 30d-30f and their electrode segments is apparent from the portions 262d, 264d, 322d, 324d, 342d, 344d, 322f, 324f, 262f and 264f of the separate conductive traces shown in Fig. 5. The capacity of each of the at least one measurement electrode (e.g., the electrode 24d) shown in Fig. 5 can then be measured between the first of the conductive traces (e.g., comprising parts 262d and 282d) and the second of the conductive traces (e.g., comprising parts 264d and 284d). Accordingly, each of the conductive traces is directly connected to a different conductive path comprised in the electrical connections 40. Put differently, each of the electrical connections 40 may comprise two conductive paths, each connected to a different of the conductive traces forming the at least one capacitance measurement electrode.

Such a configuration using capacitance measurement electrodes, each comprising or consisting of parallel conductive traces, may enable at least one of more precise capacitance measurements and improved reliability of information related to the medical flask 22d-22f retained in the associated retainer. Details of how to obtain information using the at least one capacitance measurement electrode(s) 24e-24f, 30d-30f will also be described below with reference to the processing circuitry.

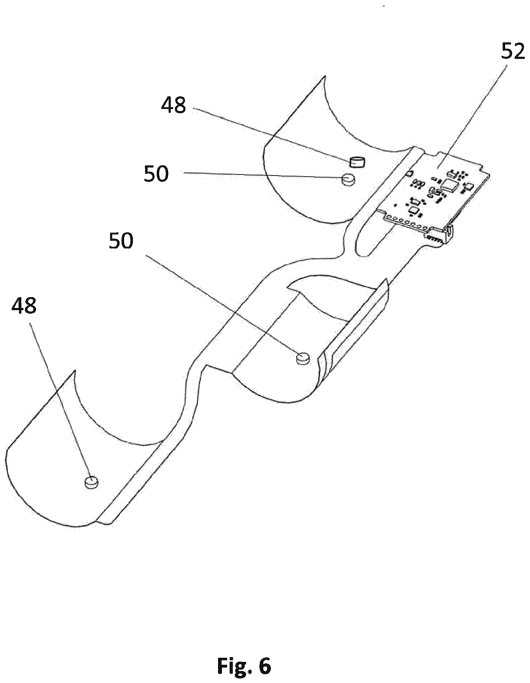

Fig. 6 shows an exemplary embodiment of the flexible circuit board 38. This flexible circuit board 38 shown in Fig. 6 may correspond to the flexible circuit board shown in one or more of Figs. 3 to 5. In addition to the at least one capacitance measurement electrode 24d-24f, 30d-30f, the at least one sensor element in this example comprises at least one of a light detection element 48 and a pressure detection element 50. The light detection element 48 may provide a spatially sensitive light measurement or an average light measurement, and may comprise one or more of a photodiode, a photoresistor or a phototransistor. The pressure detection element 50 may be a physical pressure switch or a deformation detection element such as a strain gauge. The light detection element 48 and the pressure detection element 50 may each be arranged on the flexible circuit board 38.

Such types of sensor elements 48, 50 may be low in cost and enable obtaining a sensor signal related to the medical flask 22d-22f retained by the associated retainer. For instance, the pressure detection element 50 may be used to obtain a sensor signal representative of at least one of a presence, a stiffness and a weight of the medical flask 22d, 22e retained by the associated retainer. The light detection element 48 may be used to obtain a sensor signal representative of at least one of a presence, a color, an opacity and an image of the medical flask 22d, 22f retained by the associated retainer. For example, a presence, a material and filling level of the medical flask 22d-22f may be derived based on such sensor signals, for example based on calibration values or a lookup table obtained from a memory of the processing circuitry.

As illustrated in Fig. 6, the box 2 may comprise processing circuitry 52. The processing circuitry 52 may be soldered or connected via a pin connection to the electrical connections 40 of the flexible circuit board 38 (e.g., connected to the light detection element 48, the pressure detection element 50 and the at least one capacitance measurement electrode 24e-24f, 30d-30f). The processing circuitry 50 may be encased by the compartment 36 of the (e.g., bottom portion 16 of the) flask holder 12. The light detection element 48 and the pressure detection element 50 may be arranged on a surface of the flexible surface board 38 that is opposite to a surface of the flexible circuit board 38 on which the at least one capacitance measurement electrode 24e-24f, 30d-30f is arranged.

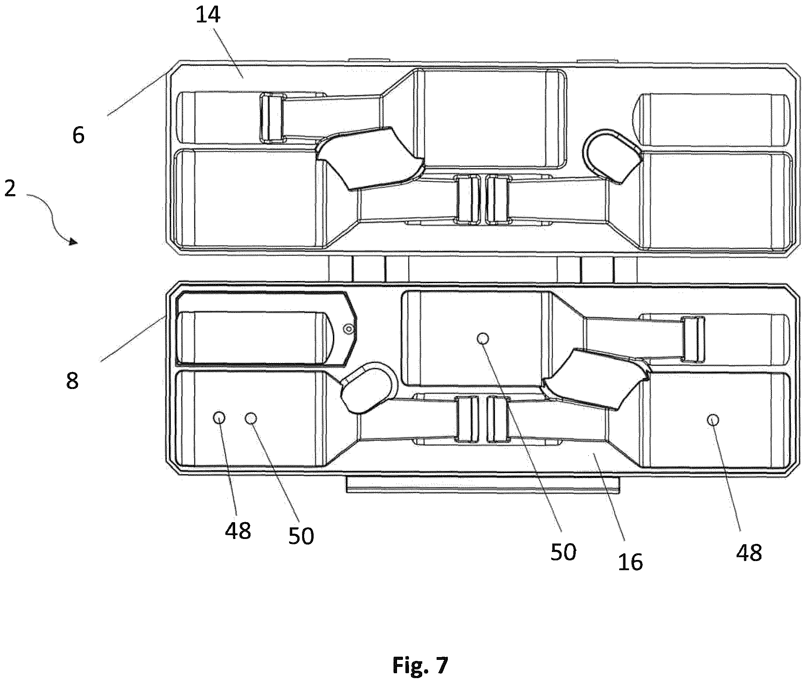

Fig. 7 shows an exemplary embodiment of the box 2. In this example, the light detection element 48 and the pressure detection element 50 are arranged such that they form parts of a wall of the retainer 12 or an outer boundary of the retainer 12. The light detection element 48 and the pressure detection element 50 are accessible via the retainer 12 or at least partially arranged inside the retainer 12. The light detection element 48 and the pressure detection element 50 may be arranged at (e.g., inside of) a hole formed in a portion of the flask holder 12 forming the retainer, for example at a hole formed in the wall section of the flask holder 12.

Such an arrangement may enable a reliable detection using the light detection element 48 and the pressure detection element 50. Furthermore, a compact arrangement enabling a plurality of measurement functions (e.g., a measurement of capacitance, a measurement of light and a measurement of pressure).

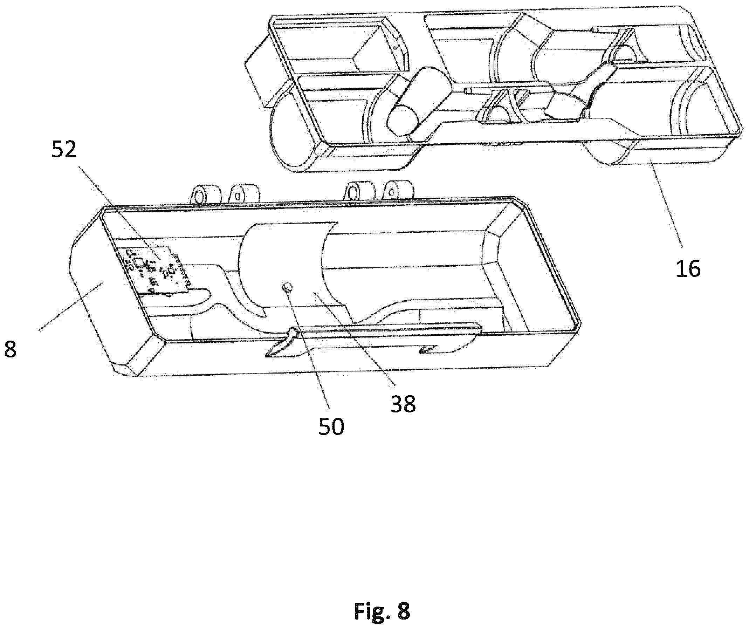

Fig. 8 shows an exemplary embodiment of parts of the box 2. As can be seen, the flask holder 12 may be detached from the housing 4. In particular, the top portion 14 may be removably attached to the upper housing part 6 and, optionally, the bottom portion 16 may be removably attached to the lower housing part 8. This enables for replacing at least one of the top portion 14 and the bottom portion 16 with a replacement part. In other words, the flask holder 12 may be replaced with a replacement holder (not shown) that may comprise retainers having different geometries and/or orientations compared with the retainers of the flask holder 12.

Put differently, the housing 4 and the flexible circuit board 38 may be used with a plurality of different flask holders 12. The present disclosure therefore provides for a system comprising the box as described herein and at least one replacement flask holder. The replacement flask holder may be configured identically to the flaks holder 12 described herein but may comprise retainers that differ therefrom in at least one of size, volume, shape and orientation. The flexible surface board 38 and the at least one sensor element (e.g., the at least one capacitance measurement electrode 24e-24f, 30d-30f) may be configured to provide the same measurement functionality for the replacement holder. For example, whilst the flask holder 12 discussed above may comprise a total of six retainers, the replacement holder may comprise a total of four retainers, each being associated with a different one of the at least one sensor element.

This may enable a user to suitably modify the box by replacing the flask holder 12 depending on the intended use. For instance, the flask holder 12 may be configured for storing six blood culture flasks of a sepsis test kit and the replacement holder may be configured for storing four syringes of a set of syringes for a blood analysis. Thus, flexibility of the box 2 may be improved.

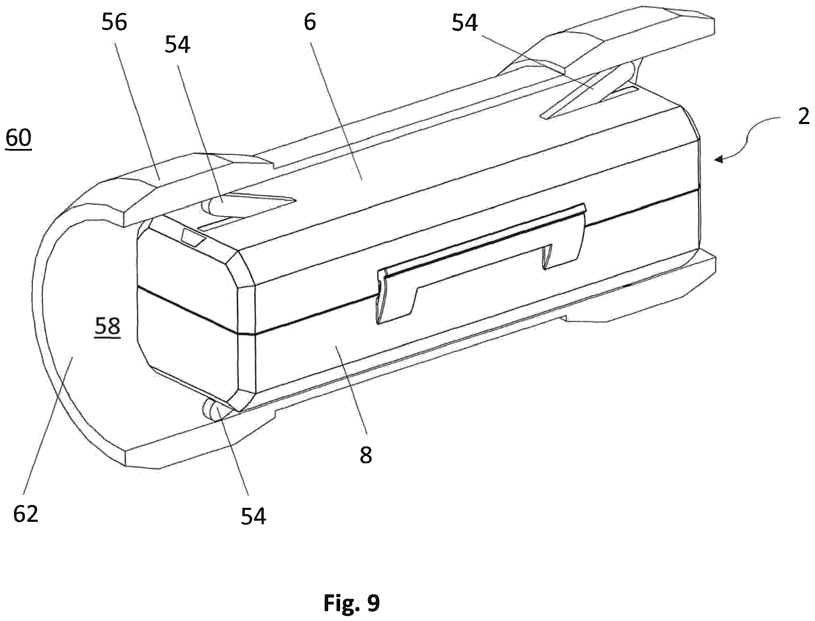

As shown in Fig. 9, the box 2 may further comprise attachment means for removably holding the box 2 inside a transport capsule 56 of a pneumatic post system when the box 2 is inserted into the transport capsule 56. In the shown example, the attachment means comprises a set of arms 54 tensioned in an outward direction of the box 2, the arms 52 being configured to hold the box 2 inside the transport capsule 56. The attachment means may provide for a damping of relative movement between the box 2 and the capsule 56.

In accordance with the present disclosure, a system comprising the box 2 and the transport capsule 56 is provided. The transport capsule 56 has an interior storage room 58 accessible from an outside 60 of the capsule 56 through a closable opening 62, wherein the housing 4 of the box 2 is shaped such that it can be completely inserted into the storage room 58 through the opening 62. The opening 62 may be sealed with a lid movably attached to or attachable to the capsule 56 (not shown).

In the following, details of the processing circuitry 52 will be discussed. The processing circuitry 52 may be configured to obtain a sensor signal using the at least one sensor element (e.g., using one or more of the electrodes 24d-24f, 30d-30f, the light detection element 48 or the pressure detection element 50) and generate, based on the sensor signal, information indicative of at least one of the medical flask (e.g., the medical flask 22d-22f) being retained by the retainer (e.g., associated with the at least one sensor element), a filling level of the medical flask when the medical flask is retained by the (e.g., associated) retainer, a material of the medical flask when the medical flask is retained by the (e.g., associated) retainer, and a type of fluid inside the medical flask when the medical flask is retained by the (e.g., associated) retainer. The processing circuitry 52 may comprise the memory and obtain at least one of a (e.g., the) lookup table or (e.g., the) calibration values from the memory, and generate the information based on the sensor signal and the obtained lookup table and/or calibration values. The processing circuitry 52 may generate the information based on a plurality of the sensor signal. For example, the plurality of the sensor signal comprise multiple sensor signals generated using different measurement parameters (e.g., different measurement frequencies, different measurement voltages, different measurement currents or else) of (e.g., each or a selection of) the at least one sensor element.

If the at least one sensor element comprises the at least one capacitance measurement electrode 24d-24f, 30d-30f, the sensor signal may be representative of a capacitance value measured using the at least one capacitance measurement electrode 24d-24f, 30d-30f. In other words, the processing circuitry 52 may measure a capacitance of the at least one capacitance measurement electrode 24d-24f, 30d-30f to obtain the sensor signal. The processing circuitry may be configured to measure a first capacitance value using the first 24d-24f of the at least one capacitance measurement electrode, measure a second capacitance value using the second 30d-30f of the at least one capacitance measurement electrode, and generate the information based on the first capacitance value and the second capacitance value. That is, the processing circuitry 52 may generate the information for the retainer comprising the first part 18d using the first electrode 24d and the second electrode 30d. The processing circuitry may generate the information for the additional retainers using the at least one measurement electrode (e.g., 24e, 24f, 30e, 30f) associated with (e.g., arranged at) each of the additional retainers.

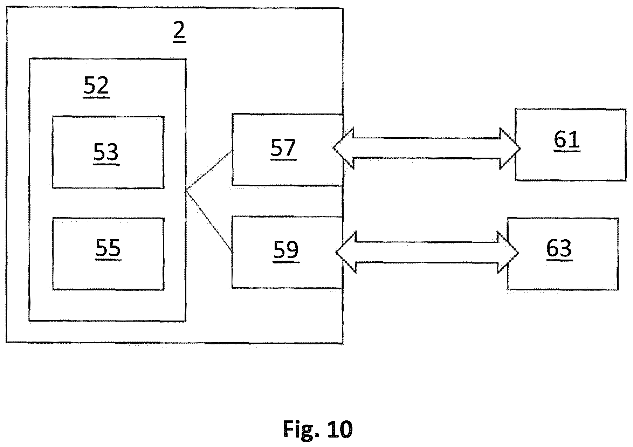

Fig. 10 shows a schematic illustration of an exemplary of a system. In this example, the processing circuitry 52 comprises a processor 53 and the memory 55, and is connected to a receiver 57 and an output interface 59 comprised in the box 2. The receiver 57 is configured to receive a wireless signal from a transmitter or sender 61. The output interface 59 is configured to output an (e.g., wireless) signal or data to an external entity 63 such as a server. The memory 55 may store instructions which, when executed by the processor 53, cause the processor 53 to perform one or more of the steps described herein. At least one of the receiver 57 and the output interface 59 may be part of the processing circuitry 52. The memory 55 may not be comprised in the processing circuitry 52 but be part of the box 2.

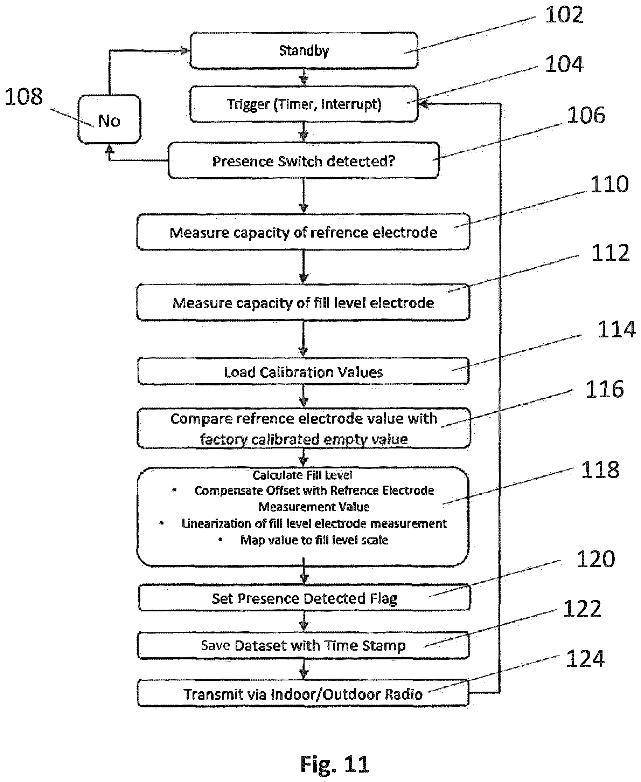

Fig. 11 shows an exemplary method that may be performed by the processing circuitry 52 (e.g., the processor 53). In a first step 102, the processing circuitry 52 is in a standby mode. In a second step 104, the processing circuitry 50 obtains a trigger such as a timer ending or an interruption signal. In response to the trigger, the processing circuitry 52 determines in step 106, using at least one of the pressure detection element 50 and the light detection element 48, whether a medical flask is retained in the retainer (e.g., the retainer comprising the first part 18d). If this is not the case (108), the processing circuitry goes back to step 102. On the other hand, if a presence of the medical flask in the retainer is detected, the method proceeds with step 110 and the processing circuitry 52 measures the capacity of the second of the at least one capacitance measurement electrode (e.g., the capacity of the electrode 30d). In step 112, the processing circuitry 52 measures the capacity of the first of the at least one capacitance measurement electrode (e.g., the capacitance of the electrode 24d).

In step 114, the calibration values are loaded from the memory 55 of the processing circuitry 52. The calibration values may be stored before shipment of the box 2 to a customer and may have been obtained by measuring the capacities of one or more of the at least one capacitance measurement electrode without an inserted flask, with different inserted flasks made of different materials, with flasks comprising different biological culture media or other different liquids, with flasks comprising different amounts of blood samples, with flasks having different filling heights and so on.

In step 116, the measured capacity of the second of the at least one capacitance measurement electrode (e.g., the capacity of the electrode 30d) measured in step 110 is compared with at least one of the loaded calibration values, for instance with a capacitance value of the second of the at least one capacitance measurement electrode that has been measured with an empty medical flask being retained by the associated retainer.

In step 118, the fill level of the medical flask (e.g., the medical flask 22d) is calculated. This may involve compensating an unwanted offset of the capacity measured in step 112 based on the capacity measured in step 110 (e.g., by subtracting the capacity measured in step 110 from the capacity measured in step 112). This may also involve linearizing the capacity measured in step 112, for example using the loaded calibration values. This may also involve mapping the capacity measured in step 112 to a fill level scale.

In step 120, information indicative of the presence of the medical flask is generated by setting a "presence detected" flag.

A dataset is generated in step 122 and (e.g., retrievably) stored together with a timestamp in the memory 55 of the processing circuitry 52.

The dataset may then be transmitted in step 124 via a wireless data communication interface, for example via the interface 59 over an indoor or outdoor radio communication link or broadcast. The method may comprise only some of the steps 102 to 124. The method may comprise additional steps not shown in Fig. 11.

The processing circuitry 52 may determine, for example in step 118, at least one of the medical flask (e.g., 22d-22f) being retained by the retainer, the filling level of the medical flask when the medical flask is retained by the retainer, a material of the medical flask when the medical flask is retained by the retainer, and a type of fluid inside the medical flask when the medical flask is retained by the retainer, based on the capacitance measurement(s) and the lookup table stored in the memory 55.

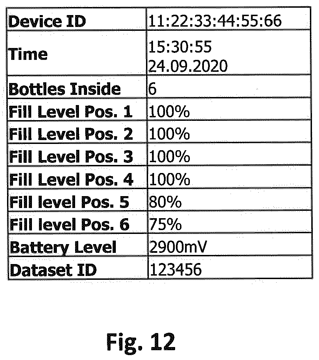

An exemplary dataset generated by the processing circuitry 52 (e.g., stored in step 122 in the memory 55) is shown in Fig. 12. As can be seen, the dataset comprises identification information of the box 2 ("Device ID"), the timestamp of generating the information contained in the dataset ("Time"), the number of medical flasks detected in the retainers ("Bottles Inside"), the filling level of each of the detected medical flasks ("Fill Level Pos.1" - "Fill Level Pos. 6"), a level of energy remaining in a battery comprised in or connected to the processing circuitry ("Battery Level") and identification information of the dataset ("Dataset ID"). The stored dataset may only comprise some of these entries or additional entries not shown in Fig. 12.

The processing circuitry 52 (e.g., the processor 53) may determine whether a user has inserted the correct number or (e.g., material) type of medical flasks, based on the dataset. In case an insufficient amount of flasks or a wrong type of flask has been inserted, the processing circuitry 52 may trigger a warning. The processing circuitry 52 may determine whether a flask has an insufficient filling height (e.g., due to a user inserting a blood sample having an insufficient volume) or a wrong liquid content, and trigger a warning. The processing circuitry 52 may send a wireless signal indicative of the warning to the external entity 63 or may trigger output of a warning signal by a warning element comprised in the box 2 (e.g., a warning light, a speaker or a vibration unit). The processing circuitry 52 may trigger a visible warning using the warning light, an auditory warning using the speaker and/or a motion alarm using the vibration unit.

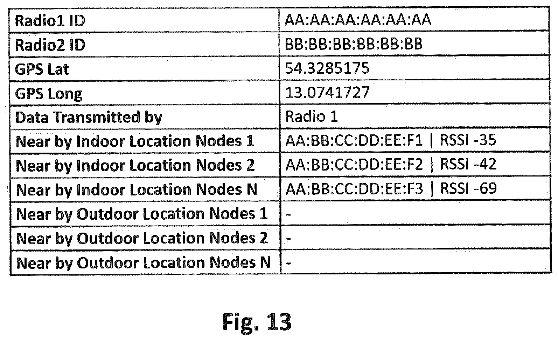

The receiver 57 is configured to receive the wireless signal, wherein the processing circuitry 52 (e.g., the processor 53) is configured to obtain, from the received wireless signal, information indicative of at least one of an identity of a sender of the wireless signal (e.g., the transmitter 61) and a location of the box 2. Such information may also be stored in the dataset described above, or in a separate dataset as shown in Fig. 13.

The dataset of Fig. 13 comprises identification information of a first radio transmitter or channel ("Radio1 ID") and of a second radio transmitter or channel ("Radio2 ID"). In the shown example, the identification information of the first transmitter is "AA:AA:AA:AA:AA:AA". Of course, this is only a general example and each letter "A" may be another letter or number. The dataset also comprises latitude and longitude position information obtained from a global positioning system, GPS ("GPS Lat", "GPS Long"), in which case the receiver may have received positioning signals from GPS satellites. In the shown example, the latitude information is "54.3285175" and the longitude information is "13.0741727". The dataset further comprises an indication ("Data Transmitted by") that data has been received by the receiver from one of the radio transmitters (e.g., the transmitter 61) or over one of the radio channels identified with the identification information. The data also comprises an indication representative of a relative position between the box 2 and one or more indoor or outdoor location nodes that transmit a signal received by the receiver of the box 2 ("Near by Indoor Location Nodes 1" - "Near by Outdoor location Nodes N"). The dataset may only comprise some of the above information, as indicated with the blank fields in Fig. 13. The dataset may comprise additional information such as a timestamp or some of the information shown in Fig. 13.

The information obtained or generated by the processing circuitry 52 (e.g., the dataset) may be retrievably stored in the memory 55 and/or output over the interface 59. This may enable the external entity 63 to obtain the information and determine a current location of the box 2. The entity 63 may determine a time-dependent location of the box 2 to track the box 2, for example using a plurality of datasets obtained from the box 2 or using a large dataset comprising indications of the position of the box 2 associated with different timestamps. In other words, the box 2 may be localized or tracked based on the information obtained or generated by the processing circuitry 50. Alternatively or additionally, the entity 63 (e.g., a server) may determine whether a user has inserted the correct number or (e.g., material) type of medical flasks, based on the dataset(s) obtained from the box 2. In case an insufficient amount of flasks or a wrong type of flask has been inserted, the entity 63 may trigger a warning. The entity 63 may determine whether a flask has an insufficient filling height (e.g., due to a user inserting a blood sample having an insufficient volume) or a wrong type of fluid content, based on the dataset(s) obtained from the box 2. In response to such a determination, the entity 63 may trigger a warning. The box 2 may be an internet-of-things, IOT, device connected to the entity 63 via the data communication interface (e.g., the interface 59). Accordingly, the box 2 provides measurement functions enabling a determination of parameters related to the medical flasks. In particular, a presence of the medical flask, a filling level or material of the medical flask, and a type of liquid inside the medical flask may be determined using the at least one sensor element. This may allow for determining whether a user has inserted an insufficient amount of medical flasks, has inserted a wrong type of medical flask, or has injected an insufficient amount of blood into a blood culture flask comprising a serum. A warning may be triggered by the box 2 or the entity 63 based on the dataset(s) generated by the processing circuitry 52, alerting the user that a mistake has been made. This may allow the user to correct the mistake (e.g., by inserting a missing flask into the box 2 or by replacing a wrong type of medical flask with a correct type), possibly reducing the time until the mistake is corrected. Handling of the medical flasks may thus be improved.

Unless mentioned otherwise, features of embodiments described herein may be combined with features of other embodiments described herein. The box 2 is not required to comprise every feature described with respect to one or more of the embodiments.

## Claims

1. A portable protective box (2) for a medical flask (22d-22f), the box (2) comprising:
a housing (4);
a flask holder (12) encased by the housing (4) and fixed relative to the housing (4), the flask holder (12) comprising a retainer configured to retain a medical flask (22d; 22e; 22f) in a stationary position relative to the housing (4); and
at least one sensor element (24d; 24e; 24f; 30d; 30e; 30f; 48; 50) having a predefined position relative to the retainer and being configured to enable detection, when the medical flask (22d; 22e; 22f) is retained by the retainer, of at least one of the medical flask (22d; 22e; 22f) being retained by the retainer, a filling level of the medical flask (22d; 22e; 22f), a material of the medical flask (22d; 22e; 22f) and a type of fluid inside the medical flask (22d; 22e; 22f), and **characterised in that**:
wherein the at least one sensor element (24d; 24e; 24f; 30d; 30e; 30f; 48; 50) comprises at least one capacitance measurement electrode (24d; 24e; 24f; 30d; 30e; 30f),
wherein the at least one capacitance measurement electrode (24d; 24e; 24f; 30d; 30e; 30f) comprises a first electrode segment (26d; 26e; 26f; 32d; 32e; 32f) and a second electrode segment (28d; 28e; 28f; 34d; 34e; 34f) contacting the first electrode segment (26d; 26e; 26f; 32d; 32e; 32f), wherein the first electrode segment (26d; 26e; 26f; 32d; 32e; 32f) is elongated in a first direction of the retainer and the second electrode segment (28d; 28e; 28f; 34d; 34e; 34f) is elongated in a second direction of the retainer different from the first direction.

2. The box (2) of claim 1, wherein
the first direction is essentially perpendicular to the second direction.

3. The box (2) of claim 1 or 2, wherein
the first direction is a longitudinal direction of the retainer and the second direction is a circumferential direction of the retainer.

4. The box (2) of any one of claims 1 to 3, further comprising
processing circuitry (52) connected to the at least one sensor element (24d; 24e; 24f; 30d; 30e; 30f; 48; 50) and configured to:
obtain a sensor signal using the at least one sensor element (24d; 24e; 24f; 30d; 30e; 30f; 48; 50); and
generate, based on the sensor signal, information indicative of at least one of:
- the medical flask (22d; 22e; 22f) being retained by the retainer;
- a filling level of the medical flask (22d; 22e; 22f) when the medical flask is retained by the retainer;
- a material of the medical flask (22d; 22e; 22f) when the medical flask is retained by the retainer; and
- a type of fluid inside the medical flask (22d; 22e; 22f) when the medical flask is retained by the retainer.

5. The box (2) of claim 4, wherein
the sensor signal is representative of a capacitance value measured using the at least one capacitance measurement electrode (24d; 24e; 24f; 30d; 30e; 30f).

6. The box (2) of claim 5, wherein
the processing circuitry (52) is configured to:
measure a first capacitance value using a first (24d; 24e; 24f) of the at least one capacitance measurement electrode (24d; 24e; 24f; 30d; 30e; 30f);
measure a second capacitance value using a second (30d; 30e; 30f) of the at least one capacitance measurement electrode (24d; 24e; 24f; 30d; 30e; 30f); and
generate the information based on the first capacitance value and the second capacitance value.

7. The box (2) of claim 6, wherein
the first (24d; 24e; 24f) of the at least one capacitance measurement electrode (24d; 24e; 24f; 30d; 30e; 30f) has a different geometry than the second (30d; 30e; 30f) of the at least one capacitance measurement electrode (24d; 24e; 24f; 30d; 30e; 30f).

8. The box (2) of any one of claims 5 to 7, further comprising
a flexible circuit board (38), wherein the at least one capacitance measurement electrode (24d; 24e; 24f; 30d; 30e; 30f) is arranged on the flexible circuit board 38 and the flexible circuit board 38 is formed such that the at least one capacitance measurement electrode (24d; 24e; 24f; 30d; 30e; 30f) conforms to a shape of at least one of the retainer and, when the medical flask (22d; 22e; 22f) is retained by the retainer, the medical flask (22d; 22e; 22f).

9. The box (2) of any one of claims 4 to 8, further comprising
a receiver (57) configured to receive a wireless signal, wherein the processing circuitry (52) is configured to:
obtain, from the received wireless signal, information indicative of at least one of an identity of a sender (61) of the wireless signal and a location of the box (2).

10. The box (2) of any one of claims 1 to 9, wherein
the at least one sensor element (24d; 24e; 24f; 30d; 30e; 30f; 48; 50) comprises at least one of a light detection element (48) and a pressure detection element (50).

11. The box of any one of claims 1 to 10, wherein
the flask holder (12) comprises a bottom portion (16) and a top portion (14) separable from the bottom portion (16), wherein a first part (18d; 18e; 18f) of the retainer is formed in the bottom portion (16) and a second part (20d; 20e; 20f) of the retainer is formed in the top portion (14).

12. The box of any one of claims 1 to 11, wherein
the flask holder (12) comprises a plurality of the retainers, each retainer being configured to retain a separate medical flask (22d; 22e; 22f) in a stationary position relative to the housing (4), and wherein
the box (2) comprises a plurality of the at least one sensor element(24d; 24e; 24f; 30d; 30e; 30f; 48; 50), each of the at least one sensor element (24d; 24e; 24f; 30d; 30e; 30f; 48; 50) having a predefined position relative to a different one of the plurality of the retainers.

13. The box (2) of any one of claims 1 to 12, further comprising
a set of arms (54) tensioned in an outward direction of the box (2), the arms (54) being configured to hold the box (2) inside a transport capsule (56) of a pneumatic post system when the box (2) is inserted into the transport capsule (56).

14. A system comprising:
the box (2) of any one of claims 1 to 13; and
a transport capsule (56) of a pneumatic post system, the transport capsule (56) having an interior storage room (58) accessible from an outside (60) of the capsule (56) through a closable opening (62), wherein the housing (4) of the box (2) is shaped such that it can be completely inserted into the storage room (58) through the opening (62).

15. A system comprising:
the box (2) of any one claims 1 to 13 or the system of claim 14, wherein the flask holder (12) is removably attached to the housing (4) and the at least one sensor element (24d; 24e; 24f; 30d; 30e; 30f; 48; 50); and
at least one replacement holder removably attachable to the housing (4) when the flask holder (12) is detached from the housing (4), the at least one replacement holder comprising a retainer configured to, when the replacement holder is removably attached to the housing (4), retain a medical flask in a stationary position relative to the housing (4), wherein the retainer of the replacement holder has a different geometry or orientation than the retainer of the flask holder (12).

16. The system of claim 15, wherein,
when the flask holder (12) is detached from the housing (4) and the replacement holder is removably attached to the housing (4), the at least one sensor element (24d; 24e; 24f; 30d; 30e; 30f; 48; 50) has a predefined position relative to the retainer of the replacement holder and is configured to enable detection of at least one of the medical flask being retained by the retainer of the replacement holder, a filling level of the medical flask, a material of the medical flask and a type of fluid inside the medical flask when the medical flask is retained by the retainer of the replacement holder.

## Patentansprüche

1. Tragbarer Schutzkasten (2) für ein medizinisches Fläschchen (22d-22f), wobei der Kasten (2) umfasst:
ein Gehäuse (4);
einen Fläschchenhalter (12), der von dem Gehäuse (4) umschlossen und in Bezug auf das Gehäuse (4) festgelegt ist, wobei der Fläschchenhalter (12) eine Halterung umfasst, die ausgebildet ist, um ein medizinisches Fläschchen (22d; 22e; 22f) in einer stationären Position in Bezug auf das Gehäuse (4) festzuhalten; und
mindestens ein Sensorelement (24d; 24e; 24f; 30d; 30e; 30f; 48; 50), das eine vorbestimmte Position in Bezug auf die Halterung aufweist und ausgebildet ist, um ein Erfassen von mindestens einem von dem medizinischen Fläschchen (22d; 22e; 22f), das von der Halterung gehalten wird, einem Füllstand des medizinischen Fläschchens (22d; 22e; 22f), einem Material des medizinischen Fläschchens (22d; 22e; 22f) und einer Art eines Fluid innerhalb des medizinischen Fläschchens (22d; 22e; 22f) zu ermöglichen, wenn das medizinische Fläschchen von der Halterung gehalten wird; und **dadurch gekennzeichnet ist, dass**:
wobei das mindestens eine Sensorelement (24d; 24e; 24f; 30d; 30e; 30f; 48; 50) mindestens eine Kapazitätsmesselektrode (24d; 24e; 24f; 30d; 30e; 30f) umfasst,
wobei die mindestens eine Kapazitätsmesselektrode (24d; 24e; 24f; 30d; 30e; 30f) ein erstes Elektrodensegment (26d; 26e; 26f; 32d; 32e; 32f) und ein zweites Elektrodensegment (28d; 28e; 28f; 34d; 34e; 34f) umfasst, das das erste Elektrodensegment (26d; 26e; 26f; 32d; 32e; 32f) kontaktiert, wobei das erste Elektrodensegment (26d; 26e; 26f; 32d; 32e; 32f) in einer ersten Richtung der Halterung langgestreckt ist und das zweite Elektrodensegment (28d; 28e; 28f; 34d; 34e; 34f) in einer von der ersten Richtung verschiedenen zweiten Richtung der Halterung langgestreckt ist.

2. Kasten (2) nach Anspruch 1, wobei
die erste Richtung im Wesentlichen senkrecht zu der zweiten Richtung ist.

3. Kasten (2) nach Anspruch 1 oder 2, wobei
die erste Richtung eine Längsrichtung der Halterung ist und die zweite Richtung eine Umfangsrichtung der Halterung ist.

4. Kasten (2) nach einem der Ansprüche 1 bis 3, ferner umfassend
eine Verarbeitungsschaltung (52), die mit dem mindestens einen Sensorelement (24d; 24e; 24f; 30d; 30e; 30f; 48; 50) verbunden und ausgebildet ist, um:
ein Sensorsignal unter Verwendung des mindestens einen Sensorelements (24d; 24e; 24f; 30d; 30e; 30f; 48; 50) zu erhalten; und
auf der Grundlage des Sensorsignals Informationen zu erzeugen, die auf mindestens eines von Folgendem hinweisen:
- das medizinische Fläschchen (22d; 22e; 22f), das von der Halterung gehalten wird;
- einen Füllstand des medizinischen Fläschchens (22d; 22e; 22f), wenn das medizinische Fläschchen von der Halterung gehalten wird;
- ein Material des medizinischen Fläschchens (22d; 22e; 22f), wenn das medizinische Fläschchen von der Halterung gehalten wird; und
- eine Art eines Fluids im Inneren des medizinischen Fläschchens (22d; 22e; 22f), wenn das medizinische Fläschchen von der Halterung gehalten wird.

5. Kasten (2) nach Anspruch 4, wobei
das Sensorsignal repräsentativ für einen Kapazitätswert ist, der unter Verwendung der mindestens einen Kapazitätsmesselektrode (24d; 24e; 24f; 30d; 30e; 30f) gemessen wird.

6. Kasten (2) nach Anspruch 5, wobei
die Verarbeitungsschaltung (52) ausgebildet ist, um:
einen ersten Kapazitätswert unter Verwendung einer ersten (24d; 24e; 24f) der mindestens einen Kapazitätsmesselektrode (24d; 24e; 24f; 30d; 30e; 30f) zu messen;
einen zweiten Kapazitätswert unter Verwendung einer zweiten (30d; 30e; 30f) der mindestens einen Kapazitätsmesselektrode (24d; 24e; 24f; 30d; 30e; 30f) messen; und
die Informationen auf der Grundlage des ersten Kapazitätswerts und des zweiten Kapazitätswerts zu erzeugen.

7. Kasten (2) nach Anspruch 6, wobei
die erste (24d; 24e; 24f) der mindestens einen Kapazitätsmesselektrode (24d; 24e; 24f; 30d; 30e; 30f) eine andere Geometrie aufweist als die zweite (30d; 30e; 30f) der mindestens einen Kapazitätsmesselektrode (24d; 24e; 24f; 30d; 30e; 30f).

8. Kasten (2) nach einem der Ansprüche 5 bis 7, ferner umfassend
eine flexible Leiterplatte (38), wobei die mindestens eine Kapazitätsmesselektrode (24d; 24e; 24f; 30d; 30e; 30f) auf der flexiblen Leiterplatte (38) angeordnet ist und die flexible Leiterplatte (38) derart geformt ist, dass die mindestens eine Kapazitätsmesselektrode (24d; 24e; 24f; 30d; 30e; 30f) einer Form von mindestens einem von der Halterung und, wenn das medizinische Fläschchen (22d; 22e; 22f) von der Halterung gehalten wird, dem medizinischen Fläschchen (22d; 22e; 22f) entspricht.

9. Kasten (2) nach einem der Ansprüche 4 bis 8, ferner umfassend
einen Empfänger (57), der ausgebildet ist, um ein drahtloses Signal zu empfangen, wobei die Verarbeitungsschaltung (52) ausgebildet ist, um:
Informationen aus dem empfangenen drahtlosen Signal zu erhalten, die mindestens auf eines von einer Identität eines Senders (61) des drahtlosen Signals und einem Standort des Kastens (2) hinweisen.

10. Kasten (2) nach einem der Ansprüche 1 bis 9, wobei
das mindestens eine Sensorelement (24d; 24e; 24f; 30d; 30e; 30f; 48; 50) mindestens eines von einem Lichterfassungselement (48) und einem Druckerfassungselement (50) umfasst.

11. Kasten nach einem der Ansprüche 1 bis 10, wobei
der Fläschchenhalter (12) einen unteren Abschnitt (16) und einen oberen Abschnitt (14) aufweist, der von dem unteren Abschnitt (16) trennbar ist, wobei ein erster Teil (18d; 18e; 18f) der Halterung in dem unteren Abschnitt (16) ausgebildet ist und ein zweiter Teil (20d; 20e; 20f) der Halterung in dem oberen Abschnitt (14) ausgebildet ist.

12. Kasten nach einem der Ansprüche 1 bis 11, wobei
der Fläschchenhalter (12) eine Vielzahl der Halterungen umfasst, wobei jede Halterung ausgebildet ist, um ein separates medizinisches Fläschchen (22d; 22e; 22f) in einer stationären Position in Bezug auf das Gehäuse (4) zu halten, und wobei
der Kasten (2) eine Vielzahl des mindestens einen Sensorelements (24d; 24e; 24f; 30d; 30e; 30f; 48; 50) umfasst, wobei jedes des mindestens einen Sensorelements (24d; 24e; 24f; 30d; 30e; 30f; 48; 50) eine vorbestimmte Position in Bezug auf eine andere der Vielzahl der Halterungen aufweist.

13. Kasten (2) nach einem der Ansprüche 1 bis 12, ferner umfassend einen Satz von Armen (54), die in eine von dem Kasten (2) nach außen gerichtete Richtung gespannt sind, wobei die Arme (54) ausgebildet sind, um den Kasten (2) innerhalb einer Transportkapsel (56) eines Rohrpostsystems zu halten, wenn der Kasten (2) in die Transportkapsel (56) eingesetzt ist.

14. System, umfassend:
den Kasten (2) nach einem der Ansprüche 1 bis 13; und
eine Transportkapsel (56) eines Rohrpostsystems, wobei die Transportkapsel (56) einen inneren Lagerraum (58) aufweist, der von einer Außenseite (60) der Kapsel (56) durch eine verschließbare Öffnung (62) zugänglich ist, wobei das Gehäuse (4) des Kastens (2) derart geformt ist, dass es durch die Öffnung (62) vollständig in den Lagerraum (58) eingesetzt werden kann.

15. System umfassend:
den Kasten (2) nach einem der Ansprüche 1 bis 13 oder das System nach Anspruch 14, wobei der Fläschchenhalter (12) lösbar am Gehäuse (4) und dem mindestens einen Sensorelement (24d; 24e; 24f; 30d; 30e; 30f; 48; 50) befestigt ist; und
mindestens einen Ersatzhalter, der lösbar an dem Gehäuse (4) angebracht werden kann, wenn der Fläschchenhalter (12) von dem Gehäuse (4) abgenommen ist, wobei der mindestens eine Ersatzhalter eine Halterung aufweist, die ausgebildet ist, um ein medizinisches Fläschchen in einer stationären Position in Bezug auf das Gehäuse (4) zu halten, wenn der Ersatzhalter lösbar an dem Gehäuse (4) angebracht ist, und wobei die Halterung des Ersatzhalters eine andere Geometrie oder Ausrichtung aufweist als die Halterung des Fläschchenhalters (12).

16. System nach Anspruch 15, wobei,
wenn der Fläschchenhalter (12) vom Gehäuse (4) gelöst ist und der Ersatzhalter lösbar am Gehäuse (4) befestigt ist, das mindestens eine Sensorelement (24d; 24e; 24f; 30d; 30e; 30f; 48; 50) eine vorbestimmte Position in Bezug auf die Halterung des Ersatzhalters aufweist und ausgebildet ist, um ein Erfassen von mindestens einem von dem medizinischen Fläschchen, das von der Halterung des Ersatzhalters gehalten wird, einem Füllstand des medizinischen Fläschchens, einem Material des medizinischen Fläschchens und einer Art eines Fluids innerhalb des medizinischen Fläschchens zu ermöglichen, wenn das medizinische Fläschchen von der Halterung des Ersatzhalters gehalten wird.

## Revendications

1. Boîte de protection portative (2) pour un flacon médical (22d-22f), la boîte (2) comprenant:
un logement (4);
un porte-flacon (12) recouvert par le logement (4) et fixé au logement (4), le porte-flacon (12) comprenant un élément de retenue conçu pour retenir un flacon médical (22d; 22e; 22f) dans une position fixe par rapport au logement (4); et
au moins un élément capteur (24d; 24e; 24f; 30d; 30e; 30f; 48; 50) ayant une position prédéfinie par rapport à l'élément de retenue et étant conçu pour permettre la détection, lorsque le flacon médical (22d; 22e; 22f) est retenu par l'élément de retenue, de l'au moins un parmi le flacon médical (22d; 22e; 22f) retenu par l'élément de retenue, un niveau de remplissage du flacon médical (22d; 22e; 22f), un matériau du flacon médical (22d; 22e; 22f) et un type de fluide à l'intérieur du flacon médical (22d; 22e; 22f), et **caractérisée en ce que**:
dans laquelle le au moins un élément capteur (24d; 24e; 24f; 30d; 30e; 30f; 48; 50) comprend au moins une électrode de mesure de capacité (24d; 24e; 24f; 30d; 30e; 30f),
dans laquelle la au moins une électrode de mesure de capacité (24d; 24e; 24f; 30d; 30e; 30f) comprend un premier segment d'électrode (26d; 26e; 26f; 32d; 32e; 32f) et un second segment d'électrode (28d; 28e; 28f; 34d; 34e; 34f) entrant en contact avec le premier segment d'électrode (26d; 26e; 26f; 32d; 32e; 32f), dans lequel le premier segment d'électrode (26d; 26e; 26f; 32d; 32e; 32f) est allongé dans une première direction de l'élément de retenue et le second segment d'électrode (28d; 28e; 28f; 34d; 34e; 34f) est allongé dans une seconde direction de l'élément de retenue différente de la première direction.

2. Boîte (2) selon la revendication 1, dans laquelle
la première direction est sensiblement perpendiculaire à la seconde direction.

3. Boîte (2) selon la revendication 1 ou 2, dans laquelle
la première direction est une direction longitudinale de l'élément de retenue et la seconde direction est une direction circonférentielle de l'élément de retenue.

4. Boîte (2) selon l'une quelconque des revendications 1 à 3, comprenant en outre
une circuiterie de traitement (52) connectée à l'au moins un élément capteur (24d; 24e; 24f; 30d; 30e; 30f; 48; 50) et conçue pour:
obtenir un signal de capteur à l'aide de l'au moins un élément capteur (24d; 24e; 24f; 30d; 30e; 30f; 48; 50); et
produire, sur la base du signal de capteur, des informations indiquant l'au moins un parmi:
- le flacon médical (22d; 22e; 22f) retenu par l'élément de retenue;
- un niveau de remplissage du flacon médical (22d; 22e; 22f) lorsque le flacon médical est retenu par l'élément de retenue;
- un matériau du flacon médical (22d; 22e; 22f) lorsque le flacon médical est retenu par l'élément de retenue; et
- un type de fluide à l'intérieur du flacon médical (22d; 22e; 22f) lorsque le flacon médical est retenu par l'élément de retenue.

5. Boîte (2) selon la revendication 4, dans laquelle
le signal de capteur représente une valeur de capacité mesurée à l'aide de l'au moins une électrode de mesure de capacité (24d; 24e; 24f; 30d; 30e; 30f).

6. Boîte (2) selon la revendication 5, dans laquelle
la circuiterie de traitement (52) est conçue pour:
mesurer une première valeur de capacité à l'aide d'une première (24d; 24e; 24f) de l'au moins une électrode de mesure de capacité (24d; 24e; 24f; 30d; 30e; 30f);
mesurer une seconde valeur de capacité à l'aide d'une seconde (30d; 30e; 30f) de l'au moins une électrode de mesure de capacité (24d; 24e; 24f; 30d; 30e; 30f); et
générer les informations sur la base de la première valeur de capacité et de la seconde valeur de capacité.

7. Boîte (2) selon la revendication 6, dans laquelle
la première (24d; 24e; 24f) de l'au moins une électrode de mesure de capacité (24d; 24e; 24f; 30d; 30e; 30f) a une géométrie différente de la seconde (30d; 30e; 30f) de l'au moins une électrode de mesure de capacité (24d; 24e; 24f; 30d; 30e; 30f).

8. Boîte (2) selon l'une quelconque des revendications 5 à 7, comprenant en outre
une carte souple de circuits imprimés (38), dans laquelle l'au moins une électrode de mesure de capacité (24d; 24e; 24f; 30d; 30e; 30f) est disposée sur la carte de circuits imprimés 38 et la carte souple de circuits imprimés 38 est formée de sorte que l'au moins une électrode de mesure de capacité (24d; 24e; 24f; 30d; 30e; 30f) épouse la forme d'au moins parmi l'élément de retenue et, lorsque le flacon médical (22d; 22e; 22f) est retenu par l'élément de retenue, le flacon médical (22d; 22e; 22f).

9. Boîte (2) selon l'une quelconque des revendications 4 à 8, comprenant en outre
un récepteur (57) conçu pour recevoir un signal sans fil, dans laquelle la circuiterie de traitement (52) est conçue pour :
obtenir, à partir du signal sans fil reçu, des informations indiquant au moins l'un parmi une identité d'un émetteur (61) du signal sans fil et un emplacement de la boîte (2).

10. Boîte (2) selon l'une quelconque des revendications 1 à 9, dans laquelle
l'au moins un élément capteur (24d; 24e; 24f; 30d; 30e; 30f; 48; 50) comprend au moins l'un parmi un élément de détection de lumière (48) et un élément de détection de pression (50).

11. Boîte selon l'une quelconque des revendications 1 à 10, dans laquelle
le porte-flacon (12) comprend une partie fond (16) et une partie supérieure (14) séparable de la partie fond (16), dans laquelle une première partie (18d; 18e; 18f) de l'élément de retenue est formée dans la partie fond (16) et une seconde partie (20d; 20e; 20f) de l'élément de retenue est formée dans la partie supérieure (14).

12. Boîte selon l'une quelconque des revendications 1 à 11, dans laquelle
le porte-flacon (12) comprend une pluralité d'éléments de retenue, chaque élément de retenue étant conçu pour retenir un flacon médical séparé (22d; 22e; 22f) dans une position fixe par rapport au logement (4), et dans laquelle
la boîte (2) comprend une pluralité de l'au moins un élément capteur (24d; 24e; 24f; 30d; 30e; 30f; 48; 50), chacun de l'au moins un élément capteur (24d; 24e; 24f; 30d; 30e; 30f; 48; 50) ayant une position prédéfinie par rapport à un élément différent de la pluralité des éléments de retenue.

13. Boîte (2) selon l'une quelconque des revendications 1 à 12, comprenant en outre
une pluralité de bras (54) tendus dans une direction extérieure de la boîte (2), les bras (54) étant conçus pour contenir la boîte (2) à l'intérieur d'une capsule de transport (56) d'un système de transport pneumatique par tube lorsque la boîte (2) est insérée dans la capsule de transport (56).

14. Système comprenant :
la boîte (2) selon l'une quelconque des revendications 1 à 13 ; et
une capsule de transport (56) d'un système de transport pneumatique par tube, la capsule de transport (56) ayant une chambre de stockage intérieure (58) accessible par l'extérieur (60) de la capsule (56) à travers une ouverture refermable (62), dans lequel le logement (4) de la boîte (2) est formé de sorte qu'elle puisse être entièrement insérée dans la chambre de stockage (58) à travers l'ouverture (62).

15. Système comprenant :
la boîte (2) selon l'une quelconque des revendications 1 à 13, dans lequel le porte-flacon (12) est fixé amovible au logement (4) et à l'au moins un élément capteur (24d; 24e; 24f; 30d; 30e; 30f; 48; 50); et
au moins un élément de retenue de remplacement pouvant être fixé amovible au logement (4) lorsque le porte-flacon (12) est détaché du logement (4), l'au moins un support de remplacement comprenant un élément de retenue conçu pour, lorsque le support de remplacement est fixé amovible au logement (4), retenir un flacon médial dans une position fixe par rapport au logement (4), dans lequel l'élément de retenue du support a une géométrie ou une orientation différente de celle de l'élément de retenue du porte-flacon (12).

16. Système selon la revendication 15, dans lequel,
lorsque le porte-flacon (12) est détaché du logement (4) et que le support de remplacement est fixé amovible au logement (4), l'au moins un élément capteur (24d; 24e; 24f; 30d; 30e; 30f; 48; 50) a une position prédéfinie par rapport à l'élément de retenue du support de remplacement et est conçu pour permettre la détection d'au moins un parmi le flacon médical retenu par l'élément de retenue du support de remplacement, un niveau de remplissage du flacon médical, un matériau du flacon médical et un type de fluide à l'intérieur du flacon médical lorsque le flacon médical est retenu par l'élément de retenue du support de remplacement.
